(19) Europäisches Patentamt
European Patent Office
Office européen des brevets

(11) Veröffentlichungsnummer : **0 415 220 B1**

# (12) EUROPÄISCHE PATENTSCHRIFT

(45) Veröffentlichungstag der Patentschrift : **02.02.94 Patentblatt 94/05**

(51) Int. Cl.$^5$ : **C07C 69/78,** C09K 19/30, C09K 19/32

(21) Anmeldenummer : **90115897.2**

(22) Anmeldetag : **20.08.90**

## (54) Chirale Dotierstoffe für Flüssigkristalle.

(30) Priorität : **01.09.89 CH 3169/89**

(43) Veröffentlichungstag der Anmeldung : **06.03.91 Patentblatt 91/10**

(45) Bekanntmachung des Hinweises auf die Patenterteilung : **02.02.94 Patentblatt 94/05**

(84) Benannte Vertragsstaaten : **CH DE FR GB IT LI NL**

(56) Entgegenhaltungen :
**DE-A- 3 333 677**
**DE-A- 3 534 780**
**DE-A- 3 633 967**

(73) Patentinhaber : **F. HOFFMANN-LA ROCHE & CO. Aktiengesellschaft**
**Postfach 3255**
**CH-4002 Basel (CH)**

(72) Erfinder : **Kelly, Stephen. Dr.**
**Salinenstrasse 3A**
**CH-4313 Möhlin (CH)**
Erfinder : **Leenhouts, Frans, Dr.**
**Kolleberglaan 33**
**B-3590 Achel (BE)**

(74) Vertreter : **Cottong, Norbert A. et al**
**Grenzacherstrasse 124 Postfach 3255**
**CH-4002 Basel (CH)**

EP 0 415 220 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue chirale Dotierstoffe für Flüssigkristalle und deren Herstellung sowie flüssigkristalline Gemische, die solche Dotierstoffe enthalten und deren Verwendung für optische und elektro-optische Zwecke.

Flüssigkristallmaterialien für elektro-optische Anzeigevorrichtungen enthalten häufig einen oder mehrere optisch aktive Zusatzstoffe zur Induzierung einer chiralen Struktur. Beispielsweise wird zur Verwendung in Anzeigevorrichtungen mit verdrillt nematischer Struktur bevorzugt ein nematischer Flüssigkristall mit optisch aktivem Zusatzstoff dotiert, z.B. um eine Umkehr der Verdrillungsrichtung (reverse twist) in TN-Zellen (twisted-nematic) zu vermeiden oder um eine ausreichende Verdrillung in Zellen mit hoch verdrillter nematischer Struktur, wie STN-Zellen (super twisted-nematic), SBE-Zellen (super birefringence effect) oder OMI-Zellen (optical mode interference) zu erreichen. Ferner können cholesterische Flüssigkristalle für Phase-Change-Zellen vorzugsweise aus einem nematischen Basismaterial und einem oder mehreren optisch aktiven Dotierstoffen bestehen und ferroelektrische Flüssigkristalle für Anzeigevorrichtungen auf der Basis von chiral getilteten smektischen Phasen können vorzugsweise aus einem Material mit getilteter smektischer Phase und einem oder mehreren optisch aktiven Dotierstoffen bestehen.

Die elektrooptischen Kennlinien von Flüssigkristall-Anzeigevorrichtungen sind temperaturabhängig, was insbesondere beim Multiplexbetrieb störend ist. Es ist jedoch bekannt, dass diese Temperaturabhängigkeit mindestens teilweise kompensiert werden kann durch Zusatz von chiralem Dotierstoff, welcher eine mit steigender Temperatur abnehmende Ganghöhe induziert. Eine solche inverse Temperaturabhängigkeit wurde nur für wenige Verbindungen gefunden. Sie kann aber auch durch Verwendung von mindestens 2 chiralen Dotierstoffen erreicht werden, welche eine unterschiedliche relative Temperaturabhängigkeit aufweisen und unterschiedliche Verdrillungsrichtung induzieren (DE-A-2827471). Allerdings erfordert dies meist einen relativ hohen Anteil an chiralen Dotierstoffen.

Cholesterische Flüssigkristalle reflektieren Licht im wesentlichen nur in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden. Ferner wurden cholesterische Flüssigkristalle auch verschiedentlich für thermochrome Anwendungen und in kosmetischen Präparaten verwendet.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Dieser technologische Hintergrund wird durch die Dokumente DE-A-36 33 967, DE-A-3534780 und DE-A-3 333 677 erläutert.

Um cholesterische Gemische mit einer Ganghöhe im Bereich der Wellenlänge des sichtbaren Lichts zu erreichen, sollten die chiralen Dotierstoffe ein möglichst hohes Verdrillungsvermögen aufweisen und in üblichen Flüssigkristallmaterialien gut löslich sein. Weiterhin sollten die chiralen Dotierstoffe eine ausreichende Stabilität besitzen, mit dem Mesophasetyp des Flüssigkristallmaterials gut kompatibel sein und den Mesophasenbereich nicht zu stark einschränken. Solche Eigenschaften wären auch für chirale Dotierstoffe zur Erzielung der eingangs genannten verdrillt nematischen Strukturen erwünscht, da ihr Anteil so niedrig gehalten werden könnte, dass die Eigenschaften des Flüssigkristallmaterials nur unwesentlich beeinflusst werden.

Die Erfindung betrifft optisch aktive Verbindungen der allgemeinen Formel

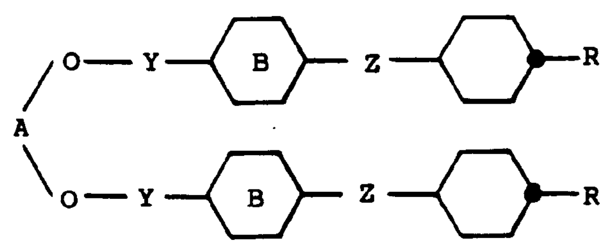

I

worin A eine optisch aktive Gruppe ausgewählt aus 1,1'-Binaphthyl-2,2'-diyl, $-CHR^1-CHR^2-$ oder $-CH(COOR^3)-CH(COOR^4)-$ darstellt; Z eine Gruppe $-(CH_2)_4-$, $-O(CH_2)_3-$ oder die trans-Form von $-OCH_2-CH=CH-$ bezeichnet; $R^1$ Wasserstoff, Methyl, Phenyl oder Cyclohexyl bedeutet; $R^2$ Methyl, Phenyl oder Cyclohexyl be-

deutet; $R^3$ und $R^4$ Alkyl bezeichnen; Y für -CO- oder $-CH_2$ - steht; Ring B 1,4-Phenylen oder trans-1,4-Cyclohexylen darstellt; und R Wasserstoff, Alkyl, Alkoxy oder Cyano bedeutet.

Die Verbindungen der Formel I zeichnen sich durch eine gute Löslichkeit und ein hohes Verdrillungsvermögen in üblichen Flüssigkristallmaterialien aus. Die Gruppen -O-Y- und insbesondere die Brückengruppen Z, welche 4 Kettenatome aufweisen, verleihen den an die optisch aktive Gruppe A gebundenen cyclischen Resten eine hohe Flexibilität. Ueberraschenderweise führt diese hohe Flexibilität jedoch nicht zu stärkerer Klärpunktsdepression, d.h. der Mesophasebereich des Flüssigkristallmaterials wird durch Zusatz von chiralen Dotierstoffen der Formel I nicht oder nur vergleichsweise wenig eingeschränkt. Die Verbindungen der Formel I sind zudem leicht herstellbar und ausreichend stabil gegen chemische Einflüsse und gegen elektrische und magnetische Felder. Sie erfüllen daher in optimaler Weise die oben genannten Anforderungen.

Die obigen Ausdrücke "Alkyl" und "Alkoxy" bezeichnen im Rahmen der vorliegenden Erfindung geradkettige oder verzweigte Reste mit vorzugsweise höchstens 12 Kohlenstoffatomen, wie Methyl, Aethyl, Propyl, Butyl, Isobutyl, 2-Methylbutyl, Pentyl, 3-Methylpentyl, Hexyl, Heptyl, Octyl, Nonyl, Decyl, Undecyl, Dodecyl, Methoxy, Aethoxy, Propyloxy, Butyloxy, Isobutyloxy, 2-Methylbutyloxy, Pentyloxy, 3-Methylpentyloxy, Hexyloxy, Heptyloxy, Octyloxy, Nonyloxy, Decyloxy, Undecyloxy, Dodecyloxy und dergleichen. Geradkettige Reste sind im allgemeinen bevorzugt. Bevorzugte Alkyl- und Alkoxyreste R sind $C_1$-$C_{12}$-Alkyl und $C_1$-$C_{12}$-Alkoxy, insbesondere $C_1$-$C_7$-Alkyl und $C_1$-$C_7$-Alkoxy, Bevorzugte Alkylreste $R^3$ und $R^4$ sind $C_1$-$C_7$-Alkyl, insbesondere $C_1$-$C_4$-Alkyl, wie Methyl, Aethyl, Propyl und Butyl.

Die optisch aktiven Gruppen A bezeichnen das Diradikal (nach Abspaltung der Hydroxygruppen) eines optisch aktiven Diols HO-A-OH. Gruppe A umfasst insbesondere die (R)-Form und die (S)-Form von 1,1'-Binaphthyl-2,2'-diyl und von $-CHR^1-CHR^2$ -, worin $R^1$ Wasserstoff bedeutet, sowie die (R,R)-Form und die (S,S)-Form von $-CH(COOR^3)-CH(COOR^4)-$ und von $-CHR^1-CHR^2$ -, worin $R^1$ von Wasserstoff verschieden ist.

Formel I umfasst je nach Bedeutung der optisch aktiven Gruppe A bzw. der Brückengruppe Z optisch aktive Verbindungen der folgenden allgemeinen Formeln

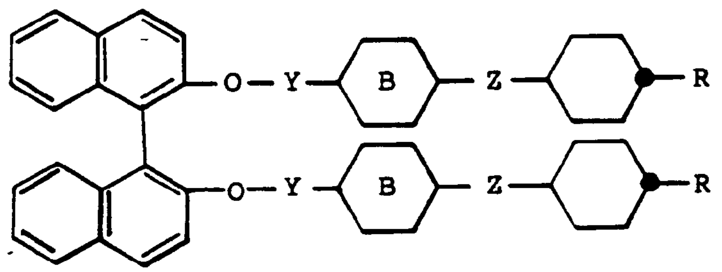

Ia

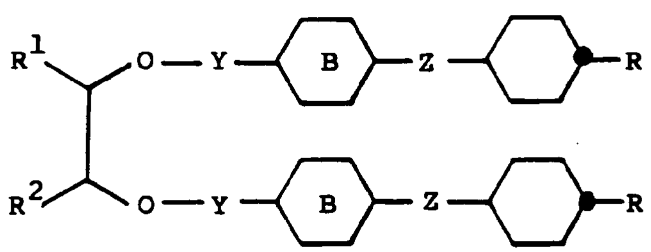

Ib

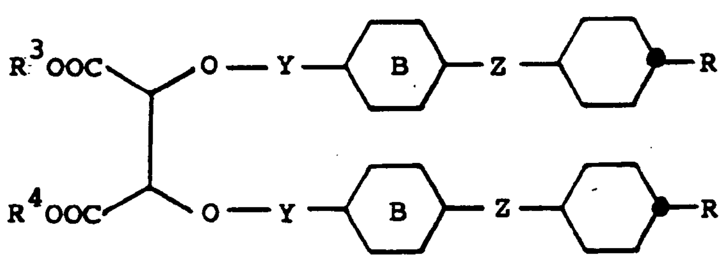

Ic

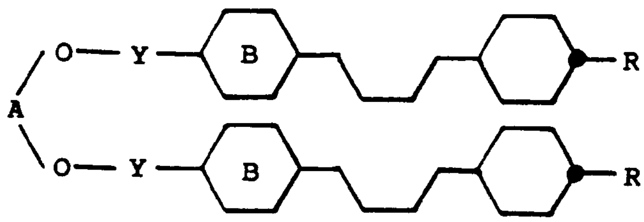

Id

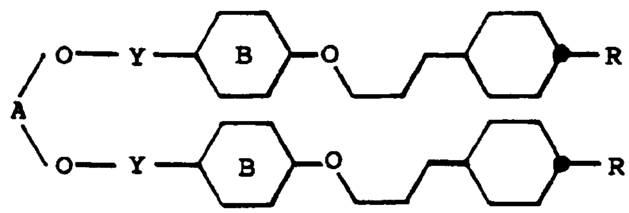

Ie

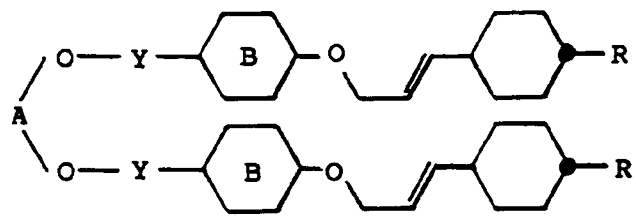

If

worin A, R, $R^1$, $R^2$, $R^3$, $R^4$, Y, Z und Ring B die obigen Bedeutungen haben.

In den obigen Formeln steht vorzugsweise $R^1$ für Wasserstoff oder für den gleichen Rest wie $R^2$.

$R^3$ und $R^4$ können gleiche oder verschiedene Bedeutung haben und bezeichnen vorzugsweise $C_1$-$C_7$-Alkyl, insbesondere $C_1$-$C_4$-Alkyl. Vorzugsweise besitzen $R^3$ und $R^4$ die gleiche Bedeutung.

Beispiele bevorzugter optisch aktiver Gruppen A sind (R)-1,1'-Binaphthyl-2,2'-diyl, (R)-1,2-Propylen, (R)-1-Phenyl-1,2-äthylen, (R)-1-Cyclohexyl-1,2-äthylen, (2R,3R)-2,3-Butylen, (1R,2R)-1,2-Diphenyl-1,2-äthylen und die Spiegelbilder dieser Gruppen [d.h. die entsprechende (S)- bzw. (S,S)-Form].

Weitere bevorzugte Gruppen A sind die (S,S)-Form und die (R,R)-Form von $-CH(COOR^3)-CH(COOR^4)-$, worin $R^3$ und $R^4$ $C_1$-$C_4$-Alkyl bedeuten.

Im allgemeinen sind diejenigen Verbindungen der obigen Formeln bevorzugt, worin R $C_1$-$C_{12}$-Alkyl oder Cyano bedeutet.

Vorzugsweise steht Ring B für 1,4-Phenylen und/oder Y für -CO-. Im allgemeinen sind diejenigen Verbindungen besonders bevorzugt, worin Z $-O(CH_2)_3-$, Y -CO- und Ring B 1,4-Phenylen bezeichnen.

Die Verbindungen der Formel I können erfindungsgemäss dadurch hergestellt werden, dass man

a) zur Herstellung der Verbindungen der Formel I, worin Y -CO- bezeichnet, ein optisch aktives Diol der allgemeinen Formel

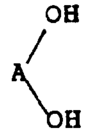

II

worin A die oben gegebene Bedeutung hat,

mit einer Carbonsäure der allgemeinen Formel

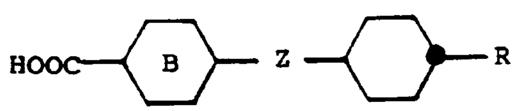

III

worin R, Z und Ring B die oben gegebenen Bedeutungen haben,
oder einem geeigneten Derivat hiervon verestert, oder
b) zur Herstellung der Verbindungen der Formel I, worin Y $-CH_2-$ bezeichnet, ein optisch aktives Diolat der allgemeinen Formel

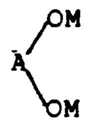

IV

worin A die oben gegebene Bedeutung hat, und M ein Alkalimetall bezeichnet,
mit einer Verbindung der allgemeinen Formel

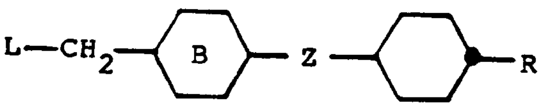

V

worin R, Z und Ring B die oben gegebenen Bedeutungen haben und L eine Abgangsgruppe bezeichnet,
veräthert.

Die Veresterung eines Diols der Formel II mit einer Carbonsäure dem Formel III oder einem geeigneten Derivat (z.B. dem Säurechlorid oder Anhydrid) kann in an sich bekannter Weise erfolgen. Eine bevorzugte Methode ist die Umsetzung des Diols und der Carbonsäure in einem inerten organischen Lösungsmittel (z.B. in einem halogenierten Kohlenwasserstoff, wie Dichlormethan) in Gegenwart von 4-(Dimethylamino)pyridin und N,N'-Dicyclohexylcarbodiimid.

Die Verätherung eines Diolates der Formel IV mit einer Verbindung der Formel V kann ebenfalls in an sich bekannter Weise, beispielsweise durch Umsetzung in einem inerten organischen Lösungsmittel, wie Toluol, Benzol, Hexan und dergleichen, bei erhöhter Temperatur erfolgen. M bezeichnet vorzugsweise Lithium, Natrium oder Kalium, insbesondere Natrium. Das Diolat kann in bekannter Weise aus dem Diol der Formel II erhalten werden, z.B. durch Umsetzung mit dem betreffenden Alkalimetall oder Alkalimetallhydrid. L bedeutet eine in $S_N2$-Reaktionen übliche Abgangsgruppe, beispielsweise Halogen, wie Chlor, Brom oder Jod, eine Sulfonyloxygruppe, wie p-Toluolsulfonyloxy und dergleichen.

Die optisch aktiven Diole der Formel II sind bekannte oder Analoge bekannter Verbindungen. Viele dieser Diole sind zudem im Handel erhältlich. Die Herstellung der Verbindungen der Formeln III und V kann nach bekannten Methoden, beispielsweise nach den in den Synthesebeispielen veranschaulichten Verfahren erfolgen.

Die Erfindung betrifft ebenfalls ein flüssigkristallines Gemisch enthaltend ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Besonders geeignet sind die Verbindungen der Formel I als chirale Dotierstoffe für nematische oder cholesterische Trägermaterialien. Das flüssigkristalline Trägermaterial kann eine Einzelverbindung oder ein Gemisch sein und besitzt vorzugsweise einen Klärpunkt von mindestens etwa 60°C, besonders bevorzugt mindestens etwa 80°C.

Der Anteil an chiralem Dotierstoff der Formel I ist im wesentlichen durch dessen Verdrillungsvermögen und die gewünschte Ganghöhe bestimmt. Der Anteil an chiralem Dotierstoff kann daher je nach Anwendung in einem breiten Bereich variieren und beispielsweise etwa 0,1-30 Gew.% betragen. Für Anzeigevorrichtungen auf der Basis von Flüssigkristallen mit verdrillt nematischer Struktur ist je nach Zellentyp und -dicke meist eine Ganghöhe von etwa 3-40 μm erforderlich und daher ein entsprechend kleiner Anteil ausreichend. Demgegenüber werden für Anwendungen, welche auf der Reflexion von sichtbarem Licht durch cholesterische Schichten beruhen, Ganghöhen von etwa 0,4-0,6 μm benötigt, was einen entsprechend höheren Anteil an chiralem Dotierstoff erfordert.

Geeignete flüssigkristalline Trägermaterialien sind in grosser Zahl bekannt und im Handel erhältlich. In der Regel sind flüssigkristalline Gemische enthaltend 2 oder mehrere Komponenten als Trägermaterialien bevorzugt. Grundsätzlich kann aber auch eine flüssigkristalline Verbindung als Trägermaterial verwendet werden, wenn sie eine ausreichend breite Mesophase aufweist.

Als Komponenten für flüssigkristalline Trägermaterialien eignen sich insbesondere Verbindungen der folgenden allgemeinen Formeln

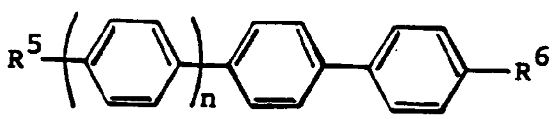

VI

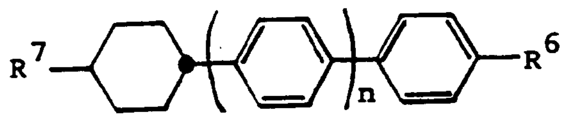

VII

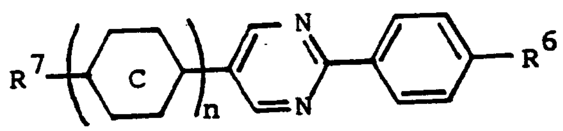

VIII

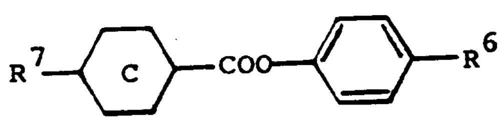

IX

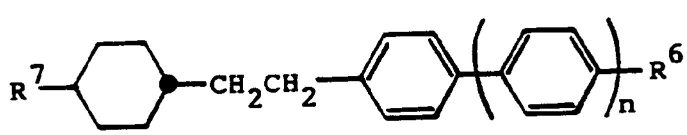

X

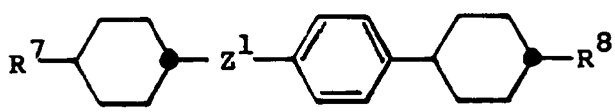

XI

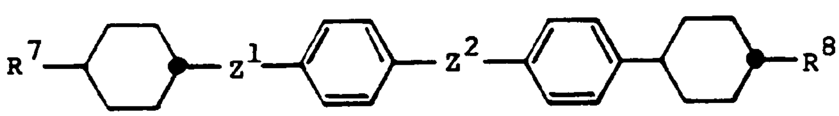

XII

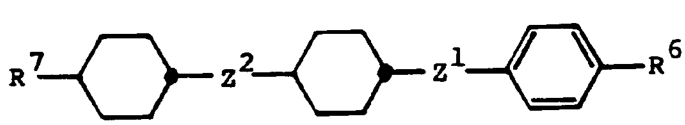

XIII

worin n für die Zahl 0 oder 1 steht; $R^5$ Alkyl, Alkoxy, Alkenyl oder Alkenyloxy bezeichnet; $R^6$ Cyano, Alkyl, Alkoxy, Alkenyl oder Alkenyloxy bedeutet; $R^7$ und $R^8$ unabhängig voneinander Alkyl oder Alkenyl bezeichnen; Ring C 1,4-Phenylen oder trans-1,4-Cyclohexylen darstellt; $Z^1$ eine einfache Kovalenzbindung, -COO- oder $-CH_2CH_2-$ bezeichnet; und $Z^2$ eine einfache Kovalenzbindung oder $-CH_2CH_2-$ bedeutet.

$R^5$, $R^6$, $R^7$ und $R^8$ besitzen vorzugsweise höchstens je 12 Kohlenstoffatome, besonders bevorzugt höchstens je 7 Kohlenstoffatome. Bevorzugte Alkenylgruppen sind 1E-Alkenyl, 3E-Alkenyl und 4Z-Alkenyl. Bevorzugte Alkenyloxygruppen sind 2E-Alkenyloxy und 3Z-Alkenyloxy.

Die Erfindung wird durch die folgenden Beispiele weiter veranschaulicht. Im Zusammenhang mit Flüssigkristallphasen und Phasenübergängen bedeuten C eine kristalline Phase, N eine nematische Phase, N* eine cholesterische Phase und I die isotrope Phase. Die Helixganghöhe wird mit p bezeichnet. Optische Antipoden besitzen jeweils "spiegelbildliche Eigenschaften", d.h. gleiche Schmelzpunkte etc., führen aber zu entgegengesetztem Helixdrehsinn und entgegengesetzter Zirkularpolarisation des reflektierten Lichts.

Beispiel 1

0,5 g 4-[4-(trans-4-Pentylcyclohexyl)-1-butyl]benzoesäure, 0,1 g R(+)-1,1'-Bi-2-naphthol und 0,04 g 4-(Dimethylamino)pyridin wurden in 50 ml Dichlormethan gelöst und die Lösung innert 10 Minuten unter Rühren portionenweise mit 0,3 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonat-Lösung und dann mit Wasser gewaschen, über Magnesiumsulfat getrocknet, filtriert und anschliessend eingeengt. Das erhaltene Rohprodukt wurde an Kieselgel mit Toluol chromatographisch gereinigt. Umkristallisation aus Aethanol ergab (R)-2,2'-Bis-[4-[4-(trans-4-pentylcyclohexyl) -1-butyl]benzoyloxy]-1,1'-binaphthyl als Oel (Smp. <25°C).

Die als Ausgangsmaterial verwendete 4-[4-(trans-4-Pentylcyclohexyl)-1-butyl]benzoesäure wurde wie folgt hergestellt:

(a) Ein Gemisch aus 20 g 3-(trans-4-Pentylcyclohexyl)-1-propanal, 66 g 4-Cyanobenzyltriphenylphosphoniumbromid und 500 ml t-Butyl-methyläther wurde unter Argonbegasung bei 0°C mit 16 g Kalium-t-butylat versetzt. Das Reaktionsgemisch wurde noch 2 Stunden bei Raumtemperatur gerührt und dann mit Wasser versetzt. Das Reaktionsgemisch wurde dreimal mit je 50 ml Diäthyläther extrahiert. Die vereinigten organischen Phasen wurden mit Wasser gewaschen, über Magnesiumsulfat getrocknet, filtriert und anschliessend eingeengt. Der Rückstand wurde mehrmals mit Hexan behandelt. Die vereinigten organischen Phasen wurden mehrmals eingeengt. Das erhaltene Rohprodukt wurde an Kieselgel mit Hexan chromatographisch gereinigt. Dies ergab 40 g 4-[4-(trans-4-Pentylcyclohexyl)-1-butenyl]benzonitril (cis/trans-Gemisch).

(b) Ein Gemisch aus 40 g 4-[4-(trans-4-Pentylcyclohexyl)-1-butenyl]benzonitril, 2 g Palladium/Kohle (5%) und 250 ml Aethylacetat wurde unter Einleitung von Wasserstoff gerührt. Anschliessend wurde das anorganische Material abfiltriert und das Filtrat eingeengt. Der Rückstand wurde an Kieselgel mit Toluol/Aethylacetat (Vol. 9:1) chromatographisch gereinigt. Umkristallisation aus Aethanol ergab 36 g reines 4-[4-(trans-4-pentylcyclohexyl)-1-butyl]benzonitril mit Smp. 51°C.

(c) Ein Gemisch von 36 g 4-[4-(trans-4-Pentylcyclohexyl)-1-butyl]benzonitril, 300 ml konzentrierter Schwefelsäure, 300 ml Eisessig und 300 ml Wasser wurde über Nacht auf eine Temperatur von 120°C erwärmt. Das gekühlte Reaktionsgemisch wurde auf Eiswasser gegossen und der weisse Niederschlag abfiltriert. Der Filterrückstand wurde mit Wasser gewaschen und anschliessend aus Aethanol umkristallisiert. Dies ergab 28 g 4-[4-(trans-4-Pentylcyclohexyl)-1-butyl]benzoesäure mit Smp. (C-N) 195°C und Klp. (N-I) 199°C.

In analoger Weise können folgende Verbindungen hergestellt werden:

(R)-2,2'-Bis-[4-[4-(trans-4-propylcyclohexyl)-1-butyl]benzoyloxy]-1,1'-binaphthyl;
(R)-2,2'-Bis-[4-[4-(trans-4-heptylcyclohexyl)-1-butyl]benzoyloxy]-1,1'-binaphthyl;
(R)-1,2-Bis-[4-[4-(trans-4-propylcyclohexyl)-1-butyl]benzoyloxy]-1-phenyläthan;
(R)-1,2-Bis-[4-[4-(trans-4-pentylcyclohexyl)-1-butyl]benzoyloxy]-1-phenyläthan, Smp. 103°C;
(R)-1,2-Bis-[4-[4-(trans-4-heptylcyclohexyl)-1-butyl]benzoyloxy]-1-phenyläthan;
(R)-1,2-Bis-[4-[4-(trans-4-propylcyclohexyl)-1-butyl]benzoyloxy]propan;
(R)-1,2-Bis-[4-[4-(trans-4-pentylcyclohexyl)-1-butyl]benzoyloxy]propan;
(R)-1,2-Bis-[4-[4-(trans-4-heptylcyclohexyl)-1-butyl]benzoyloxy]propan;
(2R,3R)-2,3-Bis-[4-[4-(trans-4-propylcyclohexyl)-1-butyl]benzoyloxy]butan;
(2R,3R)-2,3-Bis-[4-[4-(trans-4-pentylcyclohexyl)-1-butyl]benzoyloxy]butan;
(2R,3R)-2,3-Bis-[4-[4-(trans-4-heptylcyclohexyl)-1-butyl]benzoyloxy]butan;
(1R,2R)-1,2-Bis-[4-[4-(trans-4-propylcyclohexyl)-1-butyl]benzoyloxy]-1,2-diphenyläthan;
(1R,2R)-1,2-Bis-[4-[4-(trans-4-pentylcyclohexyl)-1-butyl]benzoyloxy]-1,2-diphenyläthan;
(1R,2R)-1,2-Bis-[4-[4-(trans-4-heptylcyclohexyl)-1-butyl]benzoyloxy]-1,2-diphenyläthan;
Dimethyl-(2R,3R)-2,3-Bis-[4-[4-(trans-4-pentylcyclohexyl)-1-butyl]benzoyloxy]butandioat;
Diäthyl-(2R,3R)-2,3-Bis-[4-[4-(trans-4-pentylcyclohexyl)-1-butyl]benzoyloxy]butandioat;
Dipropyl-(2R,3R)-2,3-Bis-[4-[4-(trans-4-pentylcyclohexyl)-1-butyl]benzoyloxy]butandioat;

Dibutyl-(2R,3R)-2,3-Bis-[4-[4-(trans-4-pentylcyclohexyl)-1-butyl]benzoyloxy]butandioat;
sowie die optischen Antipoden der genannten Verbindungen.

Beispiel 2

2,0 g R(+)-1,1'-Bi-2-naphthol, 5,0 g 4-[3-(trans-4-Pentylcyclohexyl)-1-propyloxy]benzoesäure, 3,0 g N,N'-Dicyclohexylcarbodiimid, 0,1 g 4-(Dimethylamino)pyridin und 500 ml Dichlormethan wurden in analoger Weise zu Beispiel 1 zu 6,5 g (R)-2,2'-Bis-[4-[3-(trans-4-pentylcyclohexyl)-1-propyloxy]benzoyloxy]-1,1'-binaphthyl mit Smp. 50°C umgesetzt.

Die als Ausgangsmaterial verwendete 4-[3-(trans-4-Pentylcyclohexyl)-1-propyloxy]benzoesäure wurde wie folgt hergestellt:

(a) Ein Gemisch von 1,9 g 4-Hydroxybenzaldehyd, 5,0 g 3-(trans-4-Pentylcyclohexyl)-1-propylbromid, 8,3 g Kaliumcarbonat und 50 ml Butanon wurde über Nacht unter Rückfluss erhitzt. Anschliessend wurde das abgekühlte Reaktionsgemisch auf Wasser gegossen und dreimal mit je 50 ml Diäthyläther extrahiert. Die vereinigten organischen Phasen wurden mit 500 ml Wasser gewaschen, über Magnesiumsulfat getrocknet, filtriert und eingeengt. Chromatographie des Rückstandes an Kieselgel mit Toluol und Umkristallisation aus Aethanol ergab 6,0 g reines 4-[3-(trans-4-Pentylcyclohexyl)-1-propyloxy]benzaldehyd.

(b) Eine Lösung von 5 g 4-[3-(trans-4-Pentylcyclohexyl)-1-propyloxy]benzaldehyd in 100 ml Aceton wurde tropfenweise mit 10 ml Jones'Reagenz versetzt. Das Gemisch wurde 1 Stunde bei Raumtemperatur gerührt und dann auf 100 ml Wasser gegossen. Der dabei entstandene Niederschlag wurde abfiltriert, portionenweise mit Wasser gewaschen, und am Vakuum getrocknet. Das Rohprodukt wurde aus Aethanol umkristallisiert und ergab 2,2 g reine 4-[3-(trans-4-Pentylcyclohexyl)-1-propyloxy]benzoesäure mit Smp. (C-N) 204°C und Klp. (N-I) 215°C.

In analoger Weise können folgende Verbindungen hergestellt werden:

(R)-2,2'-Bis-[4-[3-(trans-4-propylcyclohexyl)-1-propyloxy]-benzoyloxy]-1,1'-binaphthyl;
(R)-2,2'-Bis-[4-[3-(trans-4-heptylcyclohexyl)-1-propyloxy]-benzoyloxy]-1,1'-binaphthyl;
(2R,3R)-2,3-Bis-[4-[3-(trans-4-propylcyclohexyl)-1-propyloxy]benzoyloxy] butan;
(2R,3R)-2,3-Bis-[4-[3-(trans-4-pentylcyclohexyl)-1-propyloxy]benzoyloxy]butan, Smp. 109°C;
(2R,3R)-2,3-Bis-[4-[3-(trans-4-heptylcyclohexyl)-1-propyloxy]benzoyloxy]butan;
(1R,2R)-1,2-Bis-[4-[3-(trans-4-propylcyclohexyl)-1-propyloxy]benzoyloxy]-1,2-diphenyläthan;
(1R,2R)-1,2-Bis-[4-[3-(trans-4-pentylcyclohexyl)-1-propyloxy]benzoyloxy]-1,2-diphenyläthan, Smp. 75°C;
(1R,2R)-1,2-Bis-[4-[3-(trans-4-heptylcyclohexyl)-1-propyloxy]benzoyloxy]-1,2-diphenyläthan;
(R)-1,2-Bis-[4-[3-(trans-4-propylcyclohexyl)-1-propyloxy]benzoyloxy]-1-phenyläthan;
(R)-1,2-Bis-[4-[3-(trans-4-pentylcyclohexyl)-1-propyloxy]benzoyloxy]-1-phenyläthan, Smp. 115°C;
(R)-1,2-Bis-[4-[3-(trans-4-heptylcyclohexyl)-1-propyloxy]benzoyloxy]-1-phenyläthan;
(R)-1,2-Bis-[4-[3-(trans-4-propylcyclohexyl)-1-propyloxy]benzoyloxy]propan;
(R)-1,2-Bis-[4-[3-(trans-4-pentylcyclohexyl)-1-propyloxy]benzoyloxy]propan;
(R)-1,2-Bis-[4-[3-(trans-4-heptylcyclohexyl)-1-propyloxy]benzoyloxy]propan;
Dimethyl-(2R,3R)-2,3-Bis-[4-[3-(trans-4-pentylcyclohexyl)-1-propyloxy]benzoyloxy]butandioat;
Diäthyl-(2R,3R)-2,3-Bis-[4-[3-(trans-4-pentylcyclohexyl)-1-propyloxy]benzoyloxy]butandioat, Smp. 59°C;
Dipropyl-(2R,3R)-2,3-Bis-[4-[3-(trans-4-pentylcyclohexyl)-1-propyloxy]benzoyloxy]butandioat;
Dibutyl-(2R,3R)-2,3-Bis-[4-[3-(trans-4-pentylcyclohexyl)-1-propyloxy]benzoyloxy]butandioat, Smp. 66°C;
sowie die optischen Antipoden der genannten Verbindungen.

Beispiel 3

1,0 g R(+)-1,1'-Bi-2-naphthol, 2,5 g 4-[(E)-3-(trans-4-Pentylcyclohexyl)allyloxy]benzoesäure, 1,3 g N,N'-Dicyclohexylcarbodiimid, 0,04 g 4-(Dimethylamino)pyridin und 250 ml Dichlormethan wurden in analoger Weise zu Beispiel 1 zu 2,2 g (R)-2,2'-Bis-[4-[(E)-3-(trans-4-pentylcyclohexyl)allyloxy]benzoyloxy]-1,1'-binaphthyl mit Smp. 93°C umgesetzt.

Die als Ausgangsmaterial verwendete 4-[(E)-3-(trans-4-Pentylcyclohexyl)allyloxy]benzoesäure wurde wie folgt hergestellt:

(a) Eine Lösung von 13,5 g (E)-3-(trans-4-Pentylcyclohexyl)acrylsäure-äthylester in 100 ml Dichlormethan wurde bei -78°C und unter Stickstoffbegasung tropfenweise mit einer 20%igen Lösung von Diisobutylaluminiumhydrid in Hexan versetzt. Nach beendeter Zugabe wurde die leicht gelbe Lösung noch 1 Stunde gerührt und dann vorsichtig mit 10 ml 25%iger Salzsäure versetzt. Das Reaktionsgemisch wurde auf 100 ml Wasser gegossen und die organische Phase abgetrennt. Die wässrige Phase wurde zweimal mit je 100 ml Dichlormethan nachextrahiert. Die vereinigten organischen Phasen wurden mit 500 ml Wasser,

500 ml konzentrierter Kaliumhydrogencarbonat-Lösung und nochmals mit 500 ml Wasser gewaschen und anschliessend über Magnesiumsulfat getrocknet, filtriert und eingeengt. Destillation des Rückstandes ergab 10 g (E)-3-(trans-4-Pentylcyclohexyl)allylalkohol mit Sdp. 174-175°C/15 mmHg.

(b) Ein Gemisch von 5,0 g (E)-3-(trans-Pentylcyclohexyl)allylalkohol, 2,9 g 4-Hydroxybenzaldehyd, 4,1 g Azodicarbonsäurediäthylester, 6,2 g Triphenylphosphin und 100 ml Tetrahydrofuran wurde über Nacht bei Raumtemperatur gerührt und dann eingeengt. Der Rückstand wird in 300 ml Hexan aufgeschlämmt, filtriert und nochmals eingeengt. Chromatographie des Rückstandes an Kieselgel mit Toluol und Umkristallisation aus Hexan ergab 4,5 g reinen 4-[(E)-3-(trans-4-Pentylcyclohexyl)allyloxy]benzaldehyd mit Smp. 55°C.

(c) 4,0 g 4-[(E)-3-(trans-4-Pentylcyclohexyl)allyloxy]benzaldehyd, 10 ml Jones'Reagenz und 100 ml Aceton wurden in analoger Weise zu Beispiel 2(b) zu 2,6 g 4-[(E)-3-(trans-4-Pentylcyclohexyl)allyloxy]benzoesäure mit Smp. 215°C umgesetzt.

In analoger Weise können folgende Verbindungen hergestellt werden:

(2R,3R)-2,3-Bis(4-[(E)-3-(trans-4-propylcyclohexyl)allyloxy]benzoyloxy]butan;
(2R,3R)-2,3-Bis-[4-[(E)-3-(trans-4-pentylcyclohexyl)allyloxy]benzoyloxy]butan;
(2R,3R)-2,3-Bis-(4-[(E)-3-(trans-4-heptylcyclohexyl)allyloxy]benzoyloxy]butan;
(1R,2R)-1,2-Bis-[4-[(E)-3-(trans-4-propylcyclohexyl)allyloxy]benzoyloxy]-1,2-diphenyläthan;
(1R,2R)-1,2-Bis-[4-[(E)-3-(trans-4-pentylcyclohexyl)allyloxy]benzoyloxy]-1,2-diphenyläthan;
(1R,2R)-1,2-Bis-(4-[(E)-3-(trans-4-heptylcyclohexyl)allyloxy]benzoyloxy]-1,2-diphenyläthan;
(R)-1,2-Bis-[4-[(E)-3-(trans-4-propylcyclohexyl)allyloxy]benzoyloxy]-1-phenyläthan;
(R)-1,2-Bis-[4-[(E)-3-(trans-4-pentylcyclohexyl)allyloxy]benzoyloxy]-1-phenyläthan, Smp. 101°C;
(R)-1,2-Bis-[4-[(E)-3-(trans-4-heptylcyclohexyl)allyloxy]benzoyloxy]-1-phenyläthan;
(R)-1,2-Bis-[4-[(E)-3-(trans-4-propylcyclohexyl)allyloxy]benzoyloxy]propan;
(R)-1,2-Bis-[4-[(E)-3-(trans-4-pentylcyclohexyl)allyloxy]benzoyloxy]propan;
(R)-1,2-Bis-[4-[(E)-3-(trans-4-heptylcyclohexyl)allyloxy]benzoyloxy]propan;
Dimethyl-(2R,3R)-2,3-Bis-[-4-[(E)-3-(trans-4-pentylcyclohexyl)allyloxy]benzoyloxy]butandioat;
Diäthyl-(2R,3R)-2,3-Bis-[4-[(E)-3-(trans-4-pentylcyclohexyl)allyloxy]benzoyloxy]butandioat;
Dipropyl-(2R,3R)-2,3-Bis-[4-[(E)-3-(trans-4-pentylcyclohexyl)allyloxy]benzoyloxy]butandioat;
Dibutyl-(2R,3R)-2,3-Bis-[4-[(E)-3-(trans-4-pentylcyclohexyl)allyloxy]benzoyloxy]butandioat;

sowie die optischen Antipoden der genannten Verbindungen.

## Beispiel 4

0,5 g Natrium-R(-)-1-Phenyl-1,2-äthandiolat, 2 g 4-[4-(trans-4-Pentylcyclohexyl)-1-butyl]benzyl -p-toluolsulfonat und 100 ml Toluol wurden 48 Stunden unter Rückfluss erhitzt und dann filtriert. Das Filtrat wurde eingeengt und der Rückstand an Kieselgel mit Toluol chromatographisch gereinigt. Umkristallisation aus Aethanol ergab (R)-1,2-Bis-[4-[4-(trans-4-pentylcyclohexyl) -1-butyl]benzyloxy]-1-phenyläthan.

Das als Ausgangsmaterial verwendete 4-[4-(trans-4-Pentylcyclohexyl)-1-butyl]benzyl -p-toluolsulfonat wurde wie folgt hergestellt:

(a) Unter Argonbegasung wurden 3,8 g Lithiumaluminiumhydrid in 100 ml absolutem Tetrahydrofuran vorgelegt und innert 30 Minuten mit einer Lösung von 10 g 4-[4-(trans-4-Pentylcyclohexyl)-1-butyl]benzoesäure in 100 ml absolutem Tetrahydrofuran versetzt. Nach beendeter Zugabe wurde das Reaktionsgemisch 1 Stunde zum Rückfluss erhitzt, dann vorsichtig auf 200 ml 2N Salzsäure gegossen und dreimal mit je 100 ml Diäthyläther extrahiert. Die organischen Phasen wurden mit 100 ml gesättigter Natriumcarbonat-Lösung gewaschen, über Magnesiumsulfat getrocknet und eingeengt. Dies ergab 9,2 g 4-[4-(trans-4-Pentylcyclohexyl)-1-butyl]benzylalkohol.

(b) Eine Lösung von 9 g 4-[4-(trans-4-Pentylcyclohexyl)-1-butyl]benzylalkohol in 50 ml Pyridin wurde bei Raumtemperatur und unter Argonbegasung portionenweise mit 10 g p-Tosylchlorid versetzt. Nach 24 Stunden Rühren bei Raumtemperatur (Bildung eines weissen Niederschlages) wurde das auf 0°C gekühlte Reaktionsgemisch mit 20 ml Wasser versetzt (leicht exotherm), mit 50 ml 25%iger Salzsäure vorsichtig sauer gestellt (pH 1) und dreimal mit je 100 ml Diäthyläther extrahiert. Die vereinigten organischen Phasen wurden mit 500 ml Wasser, 500 ml verdünnter Natriumcarbonat-Lösung und nochmals mit 500 ml Wasser gewaschen und dann anschliessend über Magnesiumsulfat getrocknet und eingeengt. Dies ergab 15 g 4-[4-(trans-4-Pentylcyclohexyl)-1-butyl]benzyl-p-toluolsulfonat.

In analoger Weise können folgende Verbindungen hergestellt werden:

(R)-1,2-Bis-[4-[4-(trans-4-propylcyclohexyl)-1-butyl]benzyloxy]-1-phenyläthan;
(R)-1,2-Bis-[4-[4-(trans-4-heptylcyclohexyl)-1-butyl]benzyloxy]-1-phenyläthan;
(R)-1,2-Bis-[4-[4-(trans-4-propylcyclohexyl)-1-butyl]benzyloxy]propan;
(R)-1,2-Bis-[4-[4-(trans-4-pentylcyclohexyl)-1-butyl]benzyloxy]propan;

(R)-1,2-Bis-[4-[4-(trans-4-heptylcyclohexyl)-1-butyl]benzyloxy]propan;
(2R,3R)-2,3-Bis-[4-[4-(trans-4-propylcyclohexyl)-1-butyl]benzyloxy]butan;
(2R,3R)-2,3-Bis-[4-[4-(trans-4-pentylcyclohexyl)-1-butyl]benzyloxy]butan;
(2R,3R)-2,3-Bis-[4-[4-(trans-4-heptylcyclohexyl)-1-butyl]benzyloxy]butan;
(1R,2R)-1,2-Bis-[4-[4-(trans-4-propylcyclohexyl)-1-butyl]benzyloxy]-1,2-diphenyläthan;
(1R,2R)-1,2-Bis-[4-[4-(trans-4-pentylcyclohexyl)-1-butyl]benzyloxy]-1,2-diphenyläthan;
(1R,2R)-1,2-Bis-[4-[4-(trans-4-heptylcyclohexyl)-1-butyl]benzyloxy]-1,2-diphenyläthan;
(R)-2,2'-Bis-[4-[4-(trans-4-propylcyclohexyl)-1-butyl]benzyloxy]-1,1'-binaphthyl;
(R)-2,2'-Bis-[4-[4-(trans-4-pentylcyclohexyl)-1-butyl]benzyloxy]-1,1'-binaphthyl;
(R)-2,2'-Bis-[4-[4-(trans-4-heptylcyclohexyl)-1-butyl]benzyloxy]-1,1'-binaphthyl;
(2R,3R)-2,3-Bis-[4-[3-(trans-4-propylcyclohexyl)-1-propyloxy]benzyloxy]butan;
(2R,3R)-2,3-Bis-[4-[3-(trans-4-pentylcyclohexyl)-1-propyloxy]benzyloxy]butan;
(2R,3R)-2,3-Bis-[4-[3-(trans-4-heptylcyclohexyl)-1-propyloxy]benzyloxy]butan;
(1R,2R)-1,2-Bis-[4-[3-(trans-4-propylcyclohexyl)-1-propyloxy]benzyloxy]-1,2-diphenyläthan;
(1R,2R)-1,2-Bis-[4-[3-(trans-4-pentylcyclohexyl)-1-propyloxy]benzyloxy]-1,2-diphenyläthan;
(1R,2R)-1,2-Bis-[4-[3-(trans-4-heptylcyclohexyl)-1-propyloxy]benzyloxy]-1,2-diphenyläthan;
(R)-1,2-Bis-[4-[3-(trans-4-propylcyclohexyl)-1-propyloxy]benzyloxy]-1-phenyläthan;
(R)-1,2-Bis-[4-[3-(trans-4-pentylcyclohexyl)-1-propyloxy]benzyloxy]-1-phenyläthan;
(R)-1,2-Bis-[4-[3-(trans-4-heptylcyclohexyl)-1-propyloxy]benzyloxy]-1-phenyläthan;
(R)-1,2-Bis-[4-[3-(trans-4-propylcyclohexyl)-1-propyloxy]benzyloxy]propan;
(R)-1,2-Bis-[4-[3-(trans-4-pentylcyclohexyl)-1-propyloxy]benzyloxy]propan;
(R)-1,2-Bis-[4-[3-(trans-4-heptylcyclohexyl)-1-propyloxy]benzyloxy]propan;
(R)-2,2'-Bis-[4-[3-(trans-4-propylcyclohexyl)-1-propyloxy]benzyloxy]-1,1'-binaphthyl;
(R)-2,2'-Bis-[4-[3-(trans-4-pentylcyclohexyl)-1-propyloxy]benzyloxy]-1,1'-binaphthyl;
(R)-2,2'-Bis-[4-[3-(trans-4-heptylcyclohexyl)-1-propyloxy]benzyloxy]-1,1'-binaphthyl;
Dimethyl-(2R,3R)-2,3-Bis-[4-[4-(trans-4-pentylcyclohexyl)-1-butyl]benzyloxy]butandioat;
Diäthyl-(2R,3R)-2,3-Bis-[4-[4-(trans-4-pentylcyclohexyl)-1-butyl]benzyloxy]butandioat;
Dipropyl-(2R,3R)-2,3-Bis-[4-[4-(trans-4-pentylcyclohexyl)-1-butyl]benzyloxy]butandioat;
Dibutyl-(2R,3R)-2,3-Bis-[4-[4-(trans-4-pentylcyclohexyl)-1-butyl]benzyloxy]butandioat;
Dimethyl-(2R,3R)-2,3-Bis-[4-[3-(trans-4-pentylcyclohexyl)-1-propyloxy]benzyloxy]butandioat;
Diäthyl-(2R,3R)-2,3-Bis-[4-[3-(trans-4-pentylcyclohexyl)-1-propyloxy]benzyloxy]butandioat;
Dipropyl-(2R,3R)-2,3-Bis-[4-[3-(trans-4-pentylcyclohexyl)-1-propyloxy]benzyloxy]butandioat;
Dibutyl-(2R,3R)-2,3-Bis-[4-[3-(trans-4-pentylcyclohexyl)-1-propyloxy]benzyloxy]butandioat;
Dimethyl-(2R,3R)-2,3-Bis-[4-[(E)-3-(trans-4-pentylcyclohexyl)allyloxy]benzyloxy]butandioat;
Diäthyl-(2R,3R)-2,3-Bis-[4-[(E)-3-(trans-4-pentylcyclohexyl)allyloxy]benzyloxy]butandioat;
Dipropyl-(2R,3R)-2,3-Bis-[4-[(E)-3-(trans-4-pentylcyclohexyl)allyloxy]benzyloxy]butandioat;
Dibutyl-(2R,3R)-2,3-Bis-[4-[(E)-3-(trans-4-pentylcyclohexyl)allyloxy]benzyloxy]butandioat;

sowie die optischen Antipoden der genannten Verbindungen.

Beispiel 5

Zur Messung der induzierten Ganghöhe (Pitch) und deren Temperaturabhängigkeit in Flüssigkristallmaterialien wurde die folgende Flüssigkristall-Grundmischung BM-1 verwendet.

| | |
|---|---|
| 5,36 Gew.% | 4'-Aethyl-4-cyanobiphenyl, |
| 3,18 Gew.% | 4'-Propyl-4-cyanobiphenyl, |
| 6,08 Gew.% | 4'-Butyl-4-cyanobiphenyl, |
| 6,53 Gew.% | 4-(trans-4-Propylcyclohexyl)benzonitril, |
| 14,67 Gew.% | 4-(trans-4-Pentylcyclohexyl)benzonitril, |
| 5,21 Gew.% | 4-Aethyl-1-(trans-4-propylcyclohexyl)benzol, |
| 16,54 Gew.% | 4-Aethoxy-1-[2-(trans-4-propylcyclohexyl)äthyl]benzol, |
| 5,60 Gew.% | 4''-Pentyl-4-cyano-p-terphenyl, |
| 5,71 Gew.% | 4'-(trans-4-Pentylcyclohexyl)-4-cyanobiphenyl, |
| 15,95 Gew.% | 1-[2-(trans-4-Butylcyclohexyl)äthyl]-4-(trans-4-pentylcyclohexyl)benzol, |
| 4,74 Gew.% | 4-[2-(trans-4-Butylcyclohexyl)äthyl]-4'-(trans-4-pentylcyclohexyl)biphenyl, |
| 7,59 Gew.% | 4-[2-(trans-4-Butylcyclohexyl)äthyl]-4'-(trans-4-pentylcyclohexyl)-1,1'-äthylendibenzol, |
| 2,84 Gew.% | trans-4-[2-(trans-4-Propylcyclohexyl)äthyl]cyclohexancarbonsäure-4-cyanophenylester; |

Smp. <-30°C, Klp. (N-I) 90°C; $\Delta\varepsilon$= 8,5, $\Delta n$ = 0,139 und $\eta$ = 22 mPa·s gemessen bei 22°C.

Flüssigkristall-Grundmischung BM-1 wurde jeweils mit einem der folgenden optisch aktiven Dotierstoffe

versetzt:

D-1 = (R)-2,2'-Bis-[4-[3-(trans-4-pentylcyclohexyl)-1-propyloxy]benzoyloxy]-1,1'-binaphthyl,

D-2 = (1R,2R)-1,2-Bis-[4-[3-(trans-4-pentylcyclohexyl)-1-propyloxy]benzoyloxy]-1,2-diphenyläthan,

D-3 = (2R,3R)-2,3-Bis-[4-[3-(trans-4-pentylcyclohexyl)-1-propyloxy]benzoyloxy]butan,

D-4 = (R)-1,2-Bis-[4-[3-(trans-4-pentylcyclohexyl)-1-propyloxy]benzoyloxy]-1-phenyläthan.

Für die chiral dotierten Gemische wurden die in Tabelle 1 zusammengestellten Ergebnisse erhalten, wobei A, B und C die Parameter der Reihenentwicklung

$$\frac{1}{pc} = A + BT_1 + CT_1^2$$

bezeichnen und p, c und $T_1$ die folgenden Bedeutungen haben:

$T_1$ = T-22°C

T = Temperatur in °C

p = Ganghöhe in µm (ein positiver Wert bedeutet rechtsdrehende Helixstruktur und ein negativer Wert bedeutet linksdrehende Helixstruktur)

c = Konzentration des optisch aktiven Dotierstoffes in Gew.-%.

**Tabelle 1**

| Mischung | Dotierstoff | A $[10^{-2} \cdot \mu m^{-1} \cdot Gew.-\%^{-1}]$ | B $[10^{-4} \cdot \mu m^{-1} \cdot Gew.-\%^{-1} \cdot °C^{-1}]$ | C $[10^{-6} \cdot \mu m^{-1} \cdot Gew.-\%^{-1} \cdot °C^{-2}]$ | p•c (bei 22°C) [µm•Gew.-%] | p (bei 22°C) [µm] |
|---|---|---|---|---|---|---|
| M-1 | 0,5 Gew.-% D-1 | 55,42 | -11,4752 | -17,818 | 1,8 | 3,6 |
| M-2 | 0,3 Gew.-% D-2 | -35,73 | - 5,2971 | 4,063 | -2,8 | -9,3 |
| M-3 | 3 Gew.-% D-3 | 4,465 | - 3,705 | - 4,487 | 22,4 | 7,5 |
| M-4 | 1 Gew.-% D-4 | -21,05 | - 4,8410 | 8,080 | -4,8 | -4,8 |

Beispiel 6

Die in Tabelle 2 angegebenen Gemische M-5 und M-6 illustrieren cholesterische Mischungen, welche Licht im sichtbaren Bereich selektiv reflektieren. D-1 und D-4 bezeichnen die in Beispiel 5 angegebenen, optisch aktiven Dotierstoffe. Die Konzentrationen sind in Gew.-% angegeben.

Tabelle 2

| | M-5 | M-6 |
|---|---|---|
| 4'-Pentyl-4-cyanobiphenyl | 37,80% | 35,08% |
| 4'-Heptyl-4-cyanobiphenyl | 20,79% | 12,29% |
| 4'-Octyloxy-4-cyanobiphenyl | 13,23% | 12,28% |
| 4"-Pentyl-4-cyano-p-terphenyl | 6,62% | 6,14% |
| 4-[5-(4-Pentylphenyl)-2-pyrimidinyl]benzonitril | 4,72% | 4,39% |
| 4-[5-(trans-4-Aethylcyclohexyl)-2-pyrimidinyl]-benzonitril | 5,67% | 5,26% |
| 4-[5-(trans-4-Pentylcyclohexyl)-2-pyrimidinyl]-benzonitril | 5,67% | 5,26% |
| D-1 | 5,50% | |
| D-4 | | 12,30% |
| p (bei 22°C) | 470 nm | -550 nm |

**Patentansprüche**

1. Optisch aktive Verbindungen der allgemeinen Formel

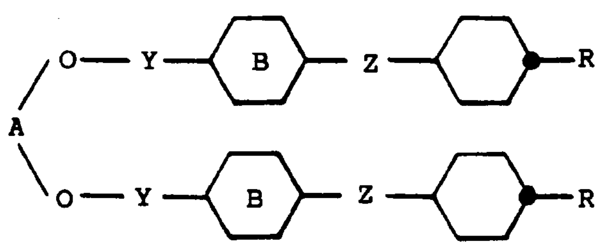

I

worin A eine optisch aktive Gruppe ausgewählt aus 1,1'-Binaphthyl-2,2'-diyl, $-CHR^1-CHR^2$ - oder $-CH(COOR^3)-CH(COOR^4)-$ darstellt; Z eine Gruppe $-(CH_2)_4-$, $-O(CH_2)_3-$ oder die trans-Form von $-OCH_2-CH{=}CH-$ bezeichnet; $R^1$ Wasserstoff, Methyl, Phenyl oder Cyclohexyl bedeutet; $R^2$ Methyl, Phenyl oder Cyclohexyl bedeutet; $R^3$ und $R^4$ Alkyl bezeichnen; Y für -CO- oder $-CH_2-$ steht; Ring B 1,4-Phenylen oder trans-1,4-Cyclohexylen darstellt; und R Wasserstoff, Alkyl, Alkoxy oder Cyano bedeutet.

2. Optisch aktive Verbindungen nach Anspruch 1, dadurch gekennzeichnet, dass A (R)-1,1'-Binaphthyl-2,2'-diyl, (R)-1,2-Propylen, (R)-1-Phenyl-1,2-äthylen, (R)-1-Cyclohexyl-1,2-äthylen, (2R,3R)-2,3-Butylen,

(1R,2R)-1,2-Diphenyl-1,2-äthylen oder das Spiegelbild einer dieser Gruppen darstellt.

3. Optisch aktive Verbindungen nach Anspruch 1, dadurch gekennzeichnet, dass A die (S,S)-Form oder die (R,R)-Form von $-CH(COOR^3)-CH(COOR^4)-$ darstellt und $R^3$ und $R^4$ $C_1$-$C_4$-Alkyl bedeuten.

4. Verbindungen nach einem der Ansprüche 1 bis 3, dadurch gekennzeichnet, dass R $C_1$-$C_{12}$-Alkyl oder Cyano bedeutet.

5. Verbindungen nach einem der Ansprüche 1 bis 4, dadurch gekennzeichnet, dass Z $-O(CH_2)_3-$, Y -CO- und Ring B 1,4-Phenylen bezeichnen.

6. Flüssigkristallines Gemisch enthaltend ein flüssigkristallines Trägermaterial und eine oder mehrere optisch aktive Verbindungen der in Anspruch 1 definierten Formel I.

7. Verfahren zur Herstellung der Verbindungen der in Anspruch 1 definierten Formel I, dadurch gekennzeichnet, dass man
a) zur Herstellung der Verbindungen der Formel I, worin Y -CO- bezeichnet, ein optisch aktives Diol der allgemeinen Formel

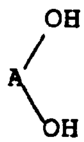

II

worin A die in Anspruch 1 gegebene Bedeutung hat,
mit einer Carbonsäure der allgemeinen Formel

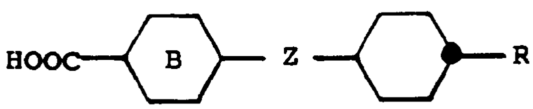

III

worin R, Z und Ring B die in Anspruch 1 gegebenen Bedeutungen haben,
oder einem geeigneten Derivat hiervon verestert, oder
b) zur Herstellung der Verbindungen der Formel I, worin Y $-CH_2-$ bezeichnet, ein optisch aktives Diolat der allgemeinen Formel

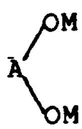

IV

worin A die in Anspruch 1 gegebene Bedeutung hat, und M ein Alkalimetall bezeichnet,
mit einer Verbindung der allgemeinen Formel

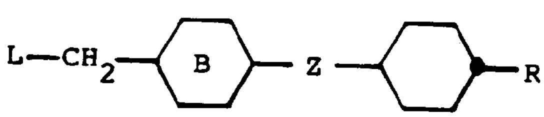

V

worin R, Z und Ring B die in Anspruch 1 gegebenen Bedeutungen haben und L eine Abgangsgruppe bezeichnet,
veräthert.

8. Verwendung flüssigkristalliner Gemische gemäss Anspruch 6 für optische und elektro-optische Zwecke.

## Claims

1. Optically active compounds of the general formula

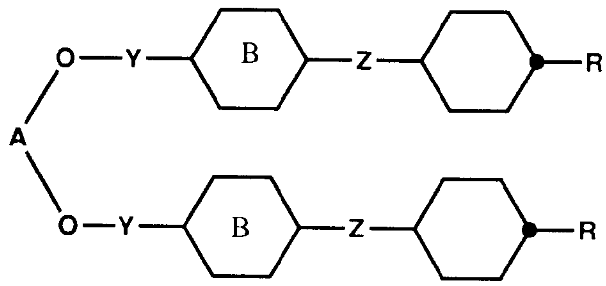

I

wherein A represents an optically active group selected from 1,1-binaphthyl-2,2-diyl, $-CHR^1-CHR^2-$ or $-CH(COOR^3)-CH(COOR^4)-$; Z denotes a group $-(CH_2)_4-$, $-O(CH_2)_3-$ or the trans form of $-OCH_2-CH=CH-$; $R^1$ signifies hydrogen, methyl, phenyl or cyclohexyl; $R^2$ signifies methyl, phenyl or cyclohexyl; $R^3$ and $R^4$ denote alkyl; Y stands for -CO- or $-CH_2-$; ring B represents 1,4-phenylene or trans-1,4-cyclohexylene; and R signifies hydrogen, alkyl, alkoxy or cyano.

2. Optically active compounds according to claim 1, characterized in that A represents (R)-1,1'-binaphthyl-2,2'-diyl, (R)-1,2-propylene, (R)-1-phenyl-1,2-ethylene, (R)-1-cyclohexyl-1,2-ethylene, (2R,3R)-2,3-butylene, (1R,2R)-1,2-diphenyl-1,2-ethylene or the mirror image of one of these groups.

3. Optically active compounds according to claim 1, characterized in that A signifies the (S,S)-form or the (R,R)-form of $-CH(COOR^3)-CH(COOR^4)-$ and $R^3$ and $R^4$ signify $C_1$-$C_4$-alkyl.

4. Compounds according to any one of claims 1 to 3, characterized in that R signifies $C_1$-$C_{12}$-alkyl or cyano.

5. Compounds according to any one of claims 1 to 4, characterized in that Z denotes $-O(CH_2)_3-$, Y denotes -CO- and ring B denotes 1,4-phenylene.

6. A liquid crystalline mixture containing a liquid crystalline carrier material and one or more optically active compounds of formula I defined in claim 1.

7. A process for the manufacture of the compounds of formula I defined in claim 1, characterized by
   a) for the manufacture of the compounds of formula I in which Y denotes -CO-, esterifying an optically active diol of the general formula

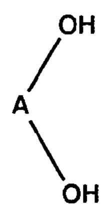

II

wherein A has the significance given in claim 1,
with a carboxylic acid of the general formula

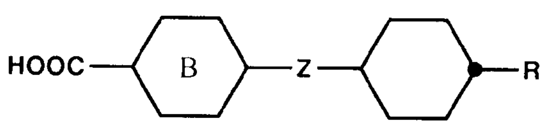

III

wherein R, Z and ring B have the significances given in claim 1,
or a suitable derivative thereof, or

b) for the manufacture of the compounds of formula I in which Y denotes $-CH_2-$, etherifying an optically active diolate of the general formula

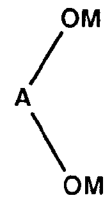

IV

wherein A has the significance given in claim 1 and M denotes an alkali metal, with a compound of the general formula

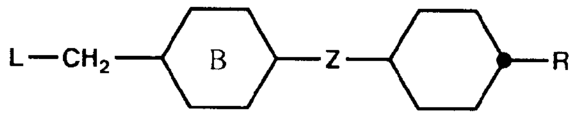

V

wherein R, Z and ring B have the significances given in claim 1 and L denotes a leaving group.

8. The use of liquid crystalline mixtures in accordance with claim 6 for optical and electro-optical purposes.

**Revendications**

1. Composés optiquement actifs de formule générale

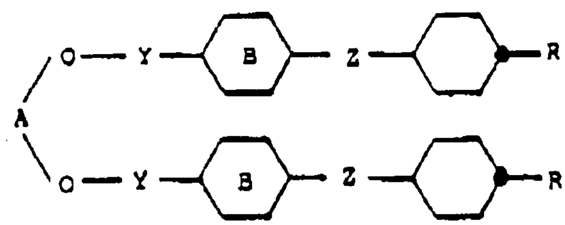

I

dans laquelle A représente un groupe optiquement actif choisi entre le 1,1'-binaphtyl-2,2'-diyle, $-CHR^1-CHR^2-$ ou $-CH(COOR^3)-CH(COOR^4)-$; Z représente un groupe $-(CH_2)_4-$, $-O(CH_2)_3-$ ou la forme trans de $-OCH_2-CH=CH-$; $R^1$ représente un hydrogène, un méthyle, un phényle ou un cyclohexyle ; $R^2$ représente un méthyle, un phényle ou un cyclohexyle ; $R^3$ et $R^4$ représentent un alcoyle ; Y représente -CO- ou $-CH_2-$ ; le noyau B représente un 1,4-phénylène ou un trans-1,4-cyclo-hexylène ; et R représente un hydrogène, un alcoyle, un alcoxy ou un cyano.

2. Composés optiquement actifs selon la revendication 1, caractérisés en ce que A représente un (R)-1,1'-binaphtyl-2,2'-diyle, un (R)-1,2-propylène, un (R)-1-phényl-1,2-éthyléne, un (R)-1-cyclohexyl-1,2-éthylène, un (2R,3R)-2,3-butylène, un (1R,2R)-1,2-diphényl-1,2-éthylène ou l'image spéculaire d'un de ces groupes.

3. Composés optiquement actifs selon la revendication 1, caractérisés en ce que A représente la forme (S,S) ou la forme (R,R) de $-CH(COOR^3)-CH(COOR^4)-$ et $R^3$ et $R^4$ représentent un alcoyle en $C_1$ à $C_4$.

4. Composés selon l'une des revendications 1 à 3, caractérisés en ce que R représente un alcoyle en $C_1$ à $C_{12}$ ou un cyano.

5. Composés selon l'une des revendications 1 à 4, caractérisés en ce que Z représente $-O(CH_2)_3-$, Y représente -CO- et le noyau B réprésente un 1,4-phénylène.

6. Mélange à cristaux liquides contenant un support à cristaux liquides et un ou plusieurs composés optiquement actifs de formule I définie dans la revendication 1.

7. Procédé de préparation des composés de formule I définie dans la revendication 1, caractérisé en ce que a) pour préparer les composés de formule I dans laquelle Y représente -CO-, on estérifie un diol optiquement actif de formule générale

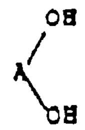

II

dans laquelle A a la signification donnée dans la revendication 1,
avec un acide carboxylique de formule générale

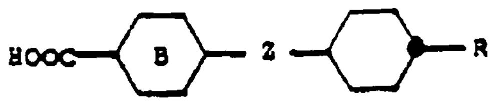

III

dans laquelle R, Z et le noyau B ont les significations données dans la revendication 1,
ou un dérivé approprié de ce corps, ou
b) pour préparer les composés de formule I dans laquelle Y représente $-CH_2-$, on éthérifie un diolate optiquement actif de formule générale

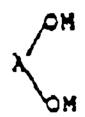

IV

dans laquelle A a la signification donnée dans la revendication 1, et M représente un métal alcalin,
avec un composé de formule générale

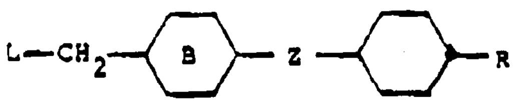

V

dans laquelle R, Z et le noyau B ont les significations données dans la revendication 1 et L représente un groupe sortant.

8. Application de mélanges à cristaux liquides selon la revendication 6 à des buts optiques et électro-optiques.